# EUROPÄISCHE PATENTSCHRIFT

(11) **EP 0 634 383 B1**
(45) Veröffentlichungstag und Bekanntmachung des Hinweises auf die Patenterteilung: **05.11.1997**
(21) Anmeldenummer: 94110535.5
(22) Anmeldetag: 06.07.1994
(51) Int. Cl.: C07C 17/087, C07C 19/08, C07C 19/12

(54) **Verfahren zur Addition von HF an halogenierte Alkene**
Process for the addition of HF to halogenated alkenes
Procédé d'addition de HF à des alkènes halogénés

(30) Priorität: 12.07.1993 DE 4323264; 19.11.1993 DE 4339539
(43) Veröffentlichungstag der Anmeldung: 18.01.1995
(73) Patentinhaber: SOLVAY (Société Anonyme), B-1050 Bruxelles (BE)
(72) Erfinder: Franz, Raimund, Dr., D-65779 Kelkheim (DE); Siegemund, Günter, Dr., D-65719 Hofheim (DE)
(74) Vertreter: Jacques, Philippe

(56) Entgegenhaltungen:
- WO-A-86/00294
- CHEMISTRY LETTERS., 1983, TOKYO JP Seiten 1135 - 1136 N. YONEDA ET AL. 'Melamine-Hydrogen Fluoride Solution. A Hihgly Effective And Convenient Hydrofluorination Reagent Of Alkenes.'

## Beschreibung

Zur Herstellung fluorhaltiger Alkane besitzt die Addition von HF an Alkene erhebliche Bedeutung. Diese Addition läßt sich jedoch vielfach nicht ohne weiteres durch Reaktion mit flüssigem oder gasförmigem HF durchführen; vielmehr treten häufig Erschwernisse und Komplikationen auf. Die Handhabung von HF ist wegen seines hohen Dampfdruckes schwierig; bei Reaktionstemperaturen oberhalb seines Siedepunktes (19,5°C) sind stets korrosionsbeständige Druckgefäße erforderlich.

Zur Umgehung oder Minderung dieser Schwierigkeit ist von G. A. Olah et al. die Verwendung von Polyhydrofluoriden des Pyridins ("Olahs Reagenz") als Hydrofluorierungs-Agentien vorgeschlagen worden (Synthesis 1973, Seite 779 bis 780). Ein ähnliches Ziel hat der Einsatz von Polyhydrofluoriden des Melamins (N. Yoneda et al., Chemistry Letters 1983, Seite 1135 bis 1136; 1984, Seite 1241 bis 1242). Diese Polyhydrofluoride enthalten 6 bis 12 Moleküle HF je Amin-Stickstoffatom, weisen jedoch einen deutlich niedrigeren Dampfdruck als reiner Fluorwasserstoff auf.

Im Vergleich zu halogenfreien Alkenen sind solche Doppelbindungen, die bereits Halogenatome tragen, in ihrer Reaktivität gegenüber HF deutlich eingeschränkt. Dies zeigt sich z. B. in der Tatsache, daß Polyhydrofluoride wie "Olahs Reagenz" oder Melamin-Polyhydrofluorid unter den von den oben erwähnten Autoren genannten Bedingungen nicht mit perhalogenierten, insbesondere perfluorierten Doppelbindungen reagieren (siehe Vergleichsbeispiel 1 und 2). Stattdessen muß die Addition von HF in diesem Fall unter drastischen Bedingungen mit reinem HF oder Alkalifluoriden durchgeführt werden. Dies führt jedoch häufig zu Halogen-Austauschreaktionen als zusätzlicher Komplikation.

Beispiele für bekannte Hydrofluorierungen unter drastischen Bedingungen sind die Umsetzung von perhalogenierten Alkenen mit HF an einem Chromoxid-Katalysator bei 200 bis 500°C, bevorzugt 300 bis 400°C, gemäß GB-PS 901 297, oder die Reaktion von Tetrafluorethen mit HF an einem Chromoxifluorid-Katalysator bei Temperaturen bis zu 200°C gemäß DE-OS 3 009 760. Ähnlich ist aus GB-PS 902 590 die Umsetzung von Hexafluorpropen mit HF zu 2-H-Heptafluorpropan bei 250 bis 450°C an Aktivkohle bekannt.

Auch beim Einsatz von wasserhaltigen oder fluorwasserstoffhaltigen Alkalifluoriden anstelle von HF (US-PS 3 130 238, US-PS 5 045 634) muß bei hohen Temperaturen gearbeitet und mit Halogenaustausch gerechnet werden. In US-PS 5 045 634 wird außerdem eine Olefinbildung durch sekundäre Abspaltung von Halogenwasserstoff, katalysiert durch das bei der Reaktion gebildete basische Alkalimetallfluorid, beschrieben. Die durch die drastischen Reaktionsbedingungen verursachte Bildung von Produktgemischen macht auch diese Verfahren ungeeignet zur rationellen Durchführung einer selektiven Addition von HF an halogenierte Alkene.

Eine bekannte, unter milderen Bedingungen verlaufende Methode ist die Umsetzung von Trifluorchlorethen CF₂=CFCl mit Kaliumfluorid in Formamid zu 1.1.1.2-Tetrafluor-chlorethan CF₃-CHFCl (W. T. Miller et al., JACS 82, Seite 3091 bis 3099 (1960)). Diese Umsetzung führt bei 55°C in 30 h zu einem Umsatz von 72 %. Die analoge Umsetzung von Hexafluorpropen führt mit 60 % Umsatz zu 2-H-Heptafluorpropan. Der Nachteil dieses Verfahrens der indirekten HF-Addition ist, daß das hierfür nötige Wasserstoff-Atom aus dem Reaktionsmedium stammt, d. h. dem Formamid, was zu unerwünschten Nebenprodukten führt.

Perfluorisobuten CF₂=C(CF₃)₂ läßt sich in Gegenwart von Ammoniumfluorid mit einer Lösung von HF in Dioxan bereits bei Raumtemperatur hydrofluorieren (I. L. Knunyants et al., Iswestija Akad. Nauk SSSR, Ser. Chim., 1965, (4), Seite 723 bis 726; in der engl. Übersetzung Seite 702 bis 704). Die Ausbeute beträgt nach dieser Literaturstelle 88 % der Theorie. Perfluorisobuten ist jedoch gegenüber anderen Perfluoralkenen durch eine extrem hohe Reaktivität gekennzeichnet (vgl. "The Chemistry of Perfluoroisobutene", I. L. Knunyants et al., Uspekhi Khimii 53, Seite 431 bis 461 (1984), in der engl. Übersetzung: Russian Chemical Reviews 53 (3), Seite 256 bis 273 (1984)). Dementsprechend ist eine Übertragung des genannten Reaktionsweges (NH₄F/HF) auf andere Perfluoralkene oder allgemein Halogenalkene in der Literatur nicht beschrieben und, wie das Vergleichsbeispiel 3 zeigt, auch nicht möglich.

Es wurde nun überraschenderweise gefunden, daß die Addition von HF an halogenierte, insbesondere fluorierte Doppelbindungen mit Hilfe von komplexen Hydrofluoriden organischer Stickstoffbasen, die jedoch im Vergleich zu den oben erwähnten Polyhydrofluoriden des Pyridins ("Olah's Reagenz") oder des Melamins relativ HF-arm sind, unter sehr milden Bedingungen erfolgt.

Gegenstand der vorliegenden Erfindung ist somit ein Verfahren zur Addition von HF an ein halogeniertes Alken der allgemeinen Formel (I)

R¹CF=CR²R³ (I)

worin R¹ bis R³ folgende Bedeutung haben:
- R¹ =: F, CF₃ oder CF₂R⁴, mit R⁴ = C₁-C₄-Alkyl, unsubstituiert oder substituiert mit einem oder mehreren Halogenatomen,
- R² =: H, Halogen oder CF₃,
- R³ =: H, F, CF₃ oder C₁-C₄-Alkyl, unsubstituiert oder substituiert mit einem oder mehreren Halogenatomen,
dadurch gekennzeichnet, daß man ein halogeniertes Alken der Formel (I) mit mindestens einem Hydrofluorid einer organischen Stickstoffbase der allgemeinen Formel (II) umsetzt,

[B·n HF] (II)

worin B eine organische Stickstoffbase und n eine ganze oder gebrochene Zahl ≤ 4 bedeutet, wobei die Umsetzung von Perfluorisobuten CF₂=C(CF₃)₂ ausgeschlossen sein soll.

Vorzugsweise setzt man als halogenierte Alkene der allgemeinen Formel (I) solche ein, bei denen
- R¹ =: F, CF₃ oder C₂F₅,
- R² =: F, Cl oder CF₃, und
- R³ =: H, F oder perfluoriertes C₁-C₄-Alkyl
bedeutet.

Insbesondere setzt man die folgenden halogenierten Alkene ein:
CF₂=CF₂, CF₂=CClF, CF₂=CFH, CF₂=CClH, CF₂=CF-CF₃, CF₃-CF=CH-CF₃, 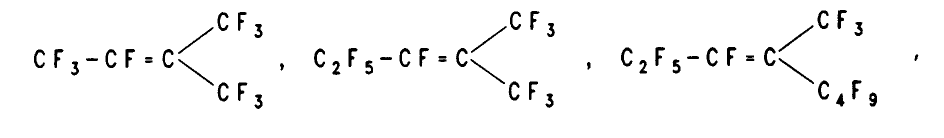 vor allem aber CF₂=CF-CF₃ (Hexafluorpropen).

Man kann bei dem erfindungsgemäßen Verfahren auch ein Gemisch aus zwei oder mehr halogenierten Alkenen der Formel (I) einsetzen.

Als Stickstoffbasen B der allgemeinen Formel (II) kommen Amine einschließlich Stickstoff-Heterocyclen infrage. Wenn man als allgemeine Formel für diese Amine die Formel (III) angibt,

R⁴R⁵R⁶N (III)

können darin die Reste R⁴, R⁵ und R⁶ gleich oder verschieden sein und
Wasserstoff,
einen Alkylrest mit 1 bis 20, vorzugsweise mit 1 bis 12, insbesondere mit 1 bis 6 C-Atomen,
einen Alkenylrest mit 2 bis 20, vorzugsweise 2 bis 12, insbesondere 2 bis 6 C-Atomen,
einen Cycloalkylrest mit 5 bis 7 C-Atomen,
einen Cycloalkenylrest mit 5 bis 7 C-Atomen,
einen Aralkylrest mit 7 bis 10 C-Atomen oder
einen Arylrest mit 6 bis 10 C-Atomen, der noch mit C₁-C₃-Alkyl oder C₁-C₃-Alkoxygruppen substituiert sein kann,
   bedeuten.

Bevorzugt sind dabei die genannten Alkyl-, Cycloalkyl, Aralkyl- und Arylreste.

Weiterhin können zwei der Reste R⁴ bis R⁶ zusammen mit dem sie tragenden N-Atom einen 5- bis 7-gliedrigen Ring bilden, welcher ein O- oder ein weiteres N-Atom enthalten kann; vorzugsweise enthält ein solcher Ring jedoch kein O- und kein weiteres N-Atom. Dieser Ring hat also 5 bis 7 Glieder, von denen eins das N-Atom und die anderen vorzugsweise CH₂-Gruppen sind. Es kann auch, was aber nicht bevorzugt ist, eine der CH₂-Gruppen durch ein O- oder N-Atom ersetzt sein.

Die Reste R⁴ bis R⁶ können auch zusammen mit dem sie tragenden N-Atom zwei oder drei 5- bis 7-gliedrige, bevorzugt gesättigte Ringe bilden, die weitere N-Atome enthalten können, wie etwa im Hexamethylentetramin oder Diazabicyclooctan.

Außerdem kann die Stickstoffbase B ein 6-gliedriger heterocyclischer Ring sein, der ein oder zwei N-Atome enthalten und auch benzokondensiert sein kann, z. B. Pyridin, Pyrimidin oder Chinolin.

Besonders bevorzugte organische Stickstoffbasen B sind tertiäre Amine, inklusive N-Heterocyclen, mit insgesamt 3 bis 12 C-Atomen, vor allem die folgenden: Trimethylamin, Triethylamin, Tri-n-propylamin, Isopropyl-diethylamin, Tri-n-butylamin, N,N-Dimethylanilin, H-Methylpiperidin, Pyridin, Chinolin, N,N'-Tetramethyl-ethylen-diamin und Hexamethylentetramin.

Die Zahl n in der allgemeinen Formel (II) bedeutet die molare Menge HF je Stickstoffatom der Base B und steht für eine ganze oder gebrochene Zahl ≤ 4, vorzugsweise 0,5 bis 3,5, insbesondere 2 bis 3.

Im folgenden werden Beispiele für die im erfindungsgemäßen Verfahren einsetzbaren, komplexen Hydrofluoride der Formel (II) angeführt:
[(CH₃)₃N · 2,8 HF]
[(C₂H₅)₃N · 2,8 HF]
[(n-C₃H₇)₃N · 3,0 HF]
[(i-C₃H₇)₂(C₂H₅)N ·2,6 HF]
[(n-C₄H₉)₃N · 2,6 HF]
[(CH₃)₂N-CH₂-CH₂-N(CH₃)₂ · 4,7] 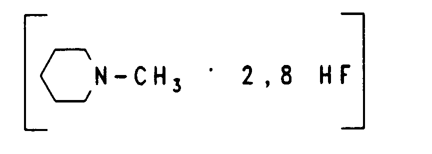 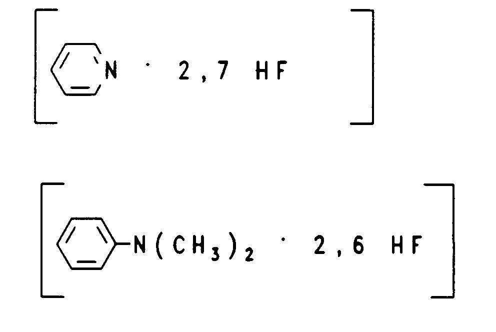 [(CH₂)₆N₄ · 2 HF]

Diese Hydrofluoride sind literaturbekannt, z. B. aus Bulletin Soc. Chim. France 1965, Seite 1890 bis 1892 oder aus J. Fluorine Chemistry 15 (1980), Seite 423 bis 434. In der angegebenen molaren Zusammensetzung handelt es sich hierbei um stabile Komplexe, die im Gegensatz zu Amin-Hydrofluoriden mit höherem Fluorwasserstoffgehalt (n > 4), wie z. B. [Pyridin · 9 HF], d. h. "Olahs Reagenz", keinen HF-Dampfdruck aufweisen, daher bedeutend einfacher zu handhaben sind und teilweise sogar in Apparaturen aus Borsilikatglas destilliert werden können. In dem erfindungsgemäßen Verfahren ist der Einsatz von [Triethylamin · 2,8 HF] oder [Tributylamin · 2,6 HF] besonders bevorzugt.

Die im erfindungsgemäßen Verfahren einzusetzenden Hydrofluoride der Formel (II) können durch direkte Umsetzung der Amine mit HF hergestellt werden. Insbesondere vor dem Hintergrund der weiter unten in den Vergleichsbeispielen 1, 2 und 4 belegten Tatsache, daß "Olahs Reagenz" und andere Amin-Hydrofluoride mit mehr als 4 HF-Molekülen je Amin-Stickstoffatom eine perhalogenierte olefinische Doppelbindung nicht zu hydrofluorieren vermögen, war es ein außerordentlich überraschender Befund, daß die im erfindungsgemäßen Verfahren einzusetzenden, vergleichsweise HF-armen Hydrofluoride sehr leicht reagieren.

Das erfindungsgemäße Verfahren kann in einem geschlossenen Druckgefäß oder bei Atmosphärendruck durchgeführt werden. Bei chargenweiser Durchführung und niedrigen Siedepunkten von Substrat und Produkt wählt man zweckmäßig einen Rührautoklaven, in welchem die Reaktion unter Eigendruck ablaufen kann. Der Fortschritt der Reaktion ist hierbei in der Regel am Abfall des Innendruckes erkennbar. Bei hinreichend hohen Siedepunkten kann ein Rührkolben, ggf. mit Rückflußkühler, zum Einsatz kommen. Nach Ende der Umsetzung kann die verbrauchte HF-Menge in geeigneter Weise, z. B. durch Einleiten, Einkondensieren oder Einpumpen von HF wieder ersetzt und eine weitere Umsetzung angeschlossen werden.

Soll die Durchführung trotz niedriger Siedepunkte drucklos erfolgen, so kann die zur Umsetzung nötige Verweilzeit des Substrats im flüssigen Hydrofluorid mittels eines Gaskreislaufes realisiert werden. Die Zufuhr des HF kann hierbei auch simultan mit der Zufuhr des Substrats erfolgen. Bevorzugt ist in diesem Fall die kontinuierliche Durchführung des Verfahrens in einer Blasensäule, die aus korrosionsbeständigem Metall, Borsilikatglas oder Kunststoff bestehen kann. Die bei dem erfindungsgemäßen Verfahren anwendbaren Reaktionstemperaturen richten sich nach dem eingesetzten halogenierten Alken und liegen im allgemeinen bei -10 bis +200°C. Man arbeitet vorzugsweise oberhalb des Schmelzpunktes des gewählten Hydrofluorids, d. h. in homogener flüssiger Phase, vorzugsweise bei 0 bis +100°C, besonders bevorzugt bei +20 bis +80°C.

Im allgemeinen ist ein Lösungsmittelzusatz nicht notwendig; bei Bedarf kann jedoch in Gegenwart hinreichender Mengen eines aprotisch polaren Lösungsmittels wie Dioxan, Tetrahydrofuran, Acetonitril oder N-Methylpyrrolidon gearbeitet werden.

Die Isolierung des Produktes der Hydrofluorierung erfolgt durch Destillation bzw. (bei Verwendung eines Druckgefäßes) durch Entspannen und Kondensieren. Es ist ein besonderer Vorteil des erfindungsgemäßen Verfahrens, daß die so hergestellten Hydrofluorierungsprodukte frei sind von Verunreinigungen, deren Bildung bei anderen Herstellungsverfahren, die bei hohen Temperaturen ablaufen, stört.

Die folgenden Beispiele erläutern das erfindungsgemäße Verfahren. Die Prozentzahlen sind Gewichtsprozente, falls nichts anderes angegeben wird.

### Vergleichsbeispiel 1

Analog einer von G. A. Olah et al., (Synthesis 1973, Seite 779 bis 783) gegebenen Vorschrift wurden in einem 500 ml-Rührautoklaven aus V4A-Stahl 100 g (0,39 Mol] des Hydrofluorids [Pyridin · 9 HF], d. h. "Olahs Reagenz", vorgelegt und 80 g Hexafluorpropen (0,53 Mol) aus einem Vorrats-Druckbehälter eingepreßt. Der Autoklaveninhalt wurde unter Rühren auf 50 bis 51°C erwärmt und 24 h auf dieser Temperatur gehalten; der Innendruck stieg während der Heizperiode von 6 auf 8,8 bar an und veränderte sich nicht mehr. Der Autoklaveninhalt wurde dann, soweit gasförmig, in einer mit Trockeneis gekühlten Falle kondensiert und analysiert. Es war kein meßbarer Gehalt an 2-H-Heptafluorpropan festzustellen.

### Vergleichsbeispiel 2

Analog einer Vorschrift von N. Yoneda et al. (Chemistry Letters 1983, Seite 1135 bis 1136] wurden in einem 500-ml-Rührautoklaven aus V4A-Stahl 160 g einer 24-proz. Lösung von Melamin (2,4,6-Triaminotriazin) in wasserfreiem Fluorwasserstoff vorgelegt (dies entspricht [Melamin · 20 HF]) und 220 g Hexafluorpropen (ca. 1,5 Mol] aus einer Vorrats-Druckflasche eingepreßt. Der Autoklaveninhalt wurde unter Rühren auf 52°C erwärmt und bei dieser Temperatur gehalten. Nach 20 h Versuchsdauer ergab die kernresonanzspektroskopische Untersuchung einer Gasprobe keinerlei Umsatz. Daraufhin wurde die Innentemperatur des Autoklaven von 52°C auf ca. 100°C erhöht, nach weiteren 20 h eine Probe entnommen und wie beschrieben analysiert. Das Spektrum ergab keinen meßbaren Umsatz zu 2-H-Heptafluorpropan.

### Vergleichsbeispiel 3

Analog der in Iswestija Akad. Nauk SSSR, Ser. Chim., 1965 (4), Seite 723 bis 726 (in der engl. Übersetzung Seite 702 bis 704) beschriebenen Umsetzung von Perfluorisobuten mit Fluorwasserstoff in Gegenwart von Ammoniumfluorid in Dioxan wurde nunmehr eine Umsetzung von Hexafluorpropen (statt Perfluorisobuten) versucht.

In einem Rührgefäß aus Polyethylen wurden 150 g wasserfreies Dioxan vorgelegt, im Eisbad gekühlt und 45 g Fluorwasserstoff (2,25 Mol) zugegeben. In einem 500-ml-Autoklaven mit Magnethubrührer wurden 3 g Ammoniumfluorid vorgelegt, in den sodann verschlossenen Autoklaven die Lösung von HF in Dioxan eingesaugt und 33 g Hexafluorpropen (0,22 Mol) eingepreßt. Die Reaktionsmischung wurde bei Raumtemperatur insgesamt 90 h gerührt. Im zeitlichen Abstand von 5 bis 10 Stunden wurden jeweils Gasproben für IR- und NMR-Analysen entnommen. Auch nach 90 h war kein 2-H-Heptafluorpropan im Reaktionsgemisch nachzuweisen. Nach dem Entspannen und Öffnen des Reaktors wurden neben unverändertem Ausgangsstoff in Dioxan gelöste, dunkelgefärbte Verharzungsprodukte gefunden.

### Vergleichsbeispiel 4

In einem 500 ml-Rührautoklaven aus V4A-Stahl wurden 126 g eines Hydrofluorids der Zusammensetzung [(n-C₄H₉)₃N · 6,6 HF] vorgelegt und 70 g Trifluorchlorethen (0,6 Mol) aus einer Vorrats-Druckflasche eingepreßt. Das Reaktionsgemisch wurde 1,5 h bei 30°C, dann 2 h ca. 60°C, schließlich 15 h bei 80°C unter Eigendruck gerührt; es wurde hierbei kein Druckabfall beobachtet. Das ¹⁹F-NMR-Spektrum einer danach gezogenen Probe zeigte keinen Umsatz zu 1.1.1.2-Tetrafluor-chlorethan.

### Vergleichsbeispiel 5

In einer mit einer Keramikfritte versehenen Gaswaschflasche wurden 90 g eines Hydrofluorids der Zusammensetzung [(n-C₄H₉)₃N · 4,6 HF] vorgelegt, bei 80°C 40 g Hexafluorpropen durchgeleitet und in einer mit Trockeneis gekühlten Falle kondensiert. Das Kondensat wurde ein weiteres Mal durch das flüssige Hydrofluorid geleitet und wiederum kondensiert. Das IR-Spektrum einer Probe zeigte das Vorliegen von reinem Hexafluorpropen an; dasselbe war der Fall nach insgesamt viermaligem Durchleiten desselben Substrats durch das genannte Hydrofluorid.

### Beispiel 1

In einem 5-Liter-Rührautoklaven wurden 1900 g (8,0 Mol) [(n-C₄H₉)₃N · 2,6 HF] vorgelegt und unter Rühren bei 25 bis 35°C 600 g Hexafluorpropen (4,0 Mol) aus einem Vorrats-Behälter eingepumpt. Anschließend wurde der Autoklav auf 75°C erwärmt und nach Erreichen dieser Temperatur über eine mit Trockeneis gekühlte Falle entspannt. Der Inhalt dieser Falle (660 g) bestand gemäß dem Infrarot- sowie den ¹H- und ¹⁹F-NMR-Spektren aus 97 % 2-H-Heptafluorpropan und ca. 3 % Hexafluorpropen. Die Zusammensetzung des Autoklaven-Rückstandes wurde nun durch Titration einer sorgfältig entgasten Probe mit Lauge ermittelt. Es wurde n = 2,1 errechnet, somit waren 0,5 x 8 = 4,0 Mol Fluorwasserstoff verbraucht worden, die nun durch Einpumpen aus einem Vorratsbehälter ersetzt wurden. Danach wurde durch Einpumpen von 600 g (4,0 Mol) Hexafluorpropen der Prozeß wiederholt. Eine 2. Wiederholung schloß sich an. Das rohe 2-H-Heptafluorpropan vom Siedepunkt -18°C wurde anschließend durch eine fraktionierte Destillation von nicht umgesetztem Hexafluorpropen befreit (Sdp. -27°C).

### Beispiel 2

In einem 500 ml-Rührkolben aus Borsilikatglas, der mit einem mit Trockeneis beschickten Rückflußkühler sowie mit einem Thermometer zur Messung der Rücklauftemperatur versehen war, wurden 157 g (1,0 Mol) [(C₂H₅)₃N · 2,8 HF] vorgelegt und aus einem Vorrats-Druckbehälter 15 g Hexafluorpropen (0,1 Mol) eingeleitet. Unter Rühren trat alsbald lebhafter Rückfluß ein, der durch schwaches Erwärmen des Kolbens aufrechterhalten wurde. Die Rückflußtemperatur stieg von anfänglich -27°C innerhalb kurzer Zeit auf - 18°C und blieb dann konstant. Das gasförmige Produkt wurde darauf über einen unterhalb des Rückflußkühlers angebrachten Hahn entnommen und in einer Kühlfalle kondensiert. Gemäß den ¹H- und ¹⁹F-NMR-Spektren bestand dieses Produkt zu 95 % aus 2-H-Heptafluorpropan und ca. 5 % Hexafluorpropen. Das Einleiten von Hexafluorpropen und die Entnahme von 2-H-Heptafluorpropan wurde viermal wiederholt.

### Vergleichsbeispiel 6

Dieser Versuch wurde analog Beispiel 2 durchgeführt, jedoch mit dem Unterschied, daß der HF-Anteil im Triethylamin-Hydrofluorid viel höher war. Es kamen 280 g einer Lösung der Zusammensetzung [(C₂H₅)₃N · 23 HF] in einer 500 ml fassenden Apparatur aus Polytetrafluorethylen zum Einsatz. Auch nach 5-stündigem Rückfließen von Hexafluorpropen war noch kein Ansteigen des Siedepunktes, somit auch kein Hinweis auf 2-H-Heptafluorpropan, festzustellen.

### Beispiel 3

Für diesen Versuch wurde ein 500-ml-Reaktionskolben aus Borsilikatglas benutzt, in dem eine Fritte zum fein verteilten Einleiten von Hexafluorpropen eingebaut war. Der Kolben war mittels eines Schlauches mit der Blase einer Apparatur zur fraktionierten Destillation verbunden, deren Abnahmeventil für gasförmiges Destillat über eine Gas-Membranpumpe wiederum mit der Fritte im Reaktionskolben verbunden war, sodaß ein Gaskreislauf zustande kam. In dem Reaktionskolben wurden nun 237 g (1,0 Mol) [(n-C₄H₉)₃N · 2,6 HF] vorgelegt. Mittels eines vor der Fritte angebrachten T-Stückes wurden im Lauf von 2 h 150 g (1,0 Mol) Hexafluorpropen aus einem Vorrats-Druckbehälter eingeleitet und nach dem Eintritt von Rückfluß in der Destillationsapparatur (-27°C) der Gaskreislauf durch Einschalten der Membranpumpe in Gang gesetzt. Dieser wurde auch nach Ende der Hexafluorpropen-Einspeisung aufrechterhalten. In der Blase der Destillationsapparatur sammelten sich im Lauf von 3 h 146 g eines siedenden Produktes, dessen Temperatur im Lauf der Reaktionszeit von -27°C auf -18°C anstieg. Dieses Produkt wurde danach entnommen und analysiert. Es bestand gemäß ¹⁹F-NMR-Spektrum aus 90 % 2-H-Hepta-fluorpropan und ca. 10 % nicht umgesetztem Hexafluorpropen.

### Beispiel 4

Eine mit Warmwasser von außen beheizbare Blasensäule aus Borsilikatglas von 22 mm Innendurchmesser und 1800 mm Länge wurde mit dem Hydrofluorid [(n-C₄H₉)₃N · 2,6 HF] gefüllt (ca. 650 ml). Bei einer Innentemperatur von 75°C wurde durch eine am Fuß der Säule angebrachte Fritte Hexafluorpropen mit einer Geschwindigkeit von 15 g/h eingeleitet. Das am Kopf der Blasensäule austretende 2-H-Heptafluorpropan wurde in einer Kühlfalle kondensiert und mittels IR-, ¹⁹F-NMR- und ¹H-NMR-Spektren identifiziert. Die Versuchsdauer betrug 4 Stunden. Die gaschromatographische Analyse dieses Rohproduktes ergab einen Gehalt von 98,4 % 2-H-Heptafluorpropan und ca. 1,5 % Hexafluorpropen.

### Beispiel 5

In der in Beispiel 4 beschriebenen Blasensäule wurde das flüssige Amin-Hydrofluorid mittels Schlauchverbindungen und einer Schlauchpumpe im Kreis geführt. In diesen Flüssigkeits-Kreislauf war ein Rührgefäß aus Polyethylen eingebaut, in welchem der verbrauchte Fluorwasserstoff durch gewichtskontrollierte Absorption aus einem Vorratsgefäß ergänzt wurde. Das ausgetriebene Rohprodukt war frei von HF-Spuren und wies einen Gehalt von fast 99 % 2-H-Heptafluorpropan und ca. 1 % Hexafluorpropen auf.

### Beispiel 6

In einem 500 ml-Rührautoklaven aus V4A-Stahl wurden 78,5 g (0,5 Mol) des Hydrofluorids [(C₂H₅)₃N · 2,8 HF] vorgelegt und 160 g Hexafluorpropen (1,07 Mol) aus einem Vorrats-Druckbehälter eingepreßt. Der Autoklaveninhalt wurde auf 50°C erwärmt und unter Eigendruck gerührt. Der Innendruck von anfänglich 10 bar verminderte sich bereits während des Aufheizens auf die Hälfte und verschwand im Lauf weiterer 4 Stunden fast völlig. Danach wurde der Autoklaveninhalt über eine Waschflasche mit 2n-Salzsäure (zur Absorption von Triethylamin) sowie einen Trockenturm mit wasserfreiem Calciumchlorid entspannt und das 2-H-Heptafluorpropan in einer mit Trockeneis gekühlten Falle kondensiert. Das so erhaltene Rohprodukt wurde gaschromatographisch sowie auch mittels GC-MS untersucht. Es enthielt neben 96,2 % des gewünschten 2-H-Heptafluorpropans ca. 3,5 % Hexafluorpropen. Eine Spurenanalyse zur Bestimmung des hochtoxischen Perfluorisobutens ergab keinen meßbaren Gehalt (<1 ppb).

### Vergleichsbeispiel 7

a) In dem in Beispiel 6 beschriebenen Autoklaven wurden 105 g einer Lösung der Zusammensetzung [(C₂H₅)₃N · 24 HF] vorgelegt, 30 g Hexafluorpropen aus einer Vorrats-Druckflasche eingepreßt und der Autoklaveninhalt auf 55°C erwärmt. Nach einstündigem Rühren bei dieser Temperatur zeigte das ¹⁹F-Spektrum einer Gasprobe keinen meßbaren Umsatz zu 2-H-Heptafluorpropan an.
b) Ein Ansatz, wie unter a) beschrieben, wurde zunächst 1 h bei 90°C, dann weitere 42 h bei 50°C unter Eigendruck gerührt. Eine danach entnommene Probe wurde analysiert. Das ¹⁹F-NMR-Spektrum zeigte abermals keinen meßbaren Umsatz zu 2-H-Heptafluorpropan an.

### Beispiel 7

In einem Rührgefäß aus Polyethylen wurden 40 g Tri-n-butylamin vorgelegt. Der Deckel des Gefäßes war über Schlauchleitungen mit einem Vorratsgefäß für Hexafluorpropen (auf einer Waage stehend), mit einem Verdampfer-Rührgefäß, welches 20 g (1 Mol) Fluorwasserstoff enthielt, sowie mit einer mit Trockeneis gekühlten Falle verbunden. In das Tributylamin wurden nunmehr Hexafluorpropen und - mittels eines in den Verdampfer geblasenen trockenen Stickstoff-Stromes - Fluorwasserstoffgas so eingeleitet, daß die im Verdampfer enthaltenen 20 g Fluorwasserstoff in der gleichen Zeit wie 150 g (1 Mol) Hexafluorpropen verbraucht waren. Der Inhalt der Kühlfalle wurde durch ein mit Calciumchlorid gefülltes Rohr umkondensiert und so von mitgerissenem Fluorwasserstoff befreit. Die gaschromatographische Analyse ergab trotz der geringen molaren Fluorwasserstoff-Menge je Amin-Molekül und trotz der extrem kurzen Verweilzeit des Hexafluorpropens im Reaktionsmedium einen Gehalt von 14 % 2-H-Heptafluorpropan neben ca. 86 % Hexafluorpropen.

### Beispiel 8

In einem 500 ml-Rührautoklaven aus rostfreiem Stahl wurden 118,5 g (0,5 Mol) des Hydrofluorids [Tri-n-butylamin · 2,6 HF] vorgelgt, 58 g Trifluorchlorethen CF₂ = CClF (0,5 Mol) aus einem Vorrats-Druckbehälter eingepreßt und das Gemisch bei 65°C gerührt. Der Innendruck fiel innerhalb von 24 h von 6 bar auf weniger als 1 bar ab. Der Autoklav wurde über eine mit Trockenes gekühlte Kühlfalle entspannt und das Produkt (Rohausbeute 61 g, 90 % der Theorie) mittels der ¹H- und ¹⁹F-NMR-Spektren als 1.1.1.2-Tetrafluor-chlorethan (R 124) identifiziert.

### Beispiel 9

In einer von außen beheizbaren Blasensäule aus Borsilikatglas vom Querschnitt 22 mm, die 1,5 m hoch mit dem flüssigen Hydrofluorid [(n-C₄H₉)₃N · 2,4 HF] gefüllt war, wurden bei einer Innentemperatur von 57°C 1,5 Liter gasförmiges Trifluorchlorethen durch eine Fritte eingeleitet und über ein Puffergefäß von ca. 1 Liter Volumen mittels einer Pumpe im Kreislauf geführt. Die Umlaufgeschwindigkeit des Gases wurde so eingestellt, daß eine gute Verteilung der Gasblasen im Medium zustande kam (5 bis 8 l/h); die Verweilzeit der Blasen im Medium betrug bei jedem Durchlauf ca. 10 Sek. Nach einer effektiven Gesamtverweilzeit des Gases im Medium von 3,5 Min. zeigte das Chromatogramm des umlaufenden Gases (Bedingungen: 5 m lange ¼"-Säule mit ®Porasil C, enthaltend 5 % Oxydipropionitril, Wärmeleitfähigkeitsdetektion, Temperatur 80°C isotherm) einen Umsatz von 50 % zu einem Produkt, welches mittels ¹H- und ¹⁹F-NMR-Spektren als 1.1.1.2-Tetrafluor-chlorethan (R 124) identifiziert wurde.

### Beispiel 10

In einer von außen beheizbaren Blasensäule aus Borsilikatglas vom Querschnitt 22 mm, die 1,5 m hoch mit dem flüssigen Hydrofluorid [(n-C₄H₉)₃N · 2,4 HF] gefüllt war, wurden bei einer Innentemperatur von 55°C 1,5 Liter gasförmiges Tetrafluorethen durch eine Fritte eingeleitet und über ein Puffergefäß von ca. 1 Liter Volumen mittels einer Pumpe im Kreislauf geführt. Die Umlaufgeschwindigkeit des Gases wurde so eingestellt, daß eine gute Verteilung der Gasblasen im Medium zustande kam (5 bis 8 l/h); die Verweilzeit der Blasen im Medium betrug bei jedem Durchlauf ca. 10 Sekunden. Nach einer effektiven Gesamtverweilzeit des Gases im Medium von 3 Minuten zeigte das Chromatogramm des umlaufenden Gases (Bedingungen wie bei Beispiel 9) einen Umsatz von 97 % zu einem Produkt, welches durch Vergleich mit einer authentischen Probe, sowie - nach Auskondensieren des Gemisches - mittels ¹H- und ¹⁹F-NMR-Spektren als Pentafluorethan (R 125) identifiziert wurde.

### Beispiel 11

In einem Rührautoklaven von 125 ml Fassungsvermögen wurden 50 g (0,21 Mol) des Hydrofluorids [(n-C₄H₉)₃N · 2,6 HF] vorgelegt und 10 g (0,1 Mol) 1.1-Difluor-chlorethen (R 1122) aus einer Vorrats-Druckflasche eingepreßt. Der Autoklaveninhalt wurde darauf 22 h bei 65°C und weitere 46 h bei 82°C unter Eigendruck gerührt. Die flüchtigen Anteile des Reaktionsgemisches wurden danach in einer Kühlfalle gesammelt und analysiert. Sie enthielten entsprechend einem Gaschromatogramm (Bedingungen wie in Beispiel 9) neben Trifluorchlorethen einen Anteil von 12 %, der mittels ¹H- und ¹⁹F-Kernresonanzspektren als 1.1.1-Trifluor-chlorethen (R 133a) identifiziert wurde.

### Beispiel 12

In einem Rührautoklaven von 125 ml Fassungsvermögen wurden 30 g (0,13 Mol) des Hydrofluorids [(n-C₄H₉)₃N · 2,6 HF] vorgelegt und 11 g (0,06 Mol) 2-H-Heptafluor-2-buten aus einer Vorrats-Druckflasche eingepreßt. Das Reaktionsgemisch wurde bei 65°C gerührt, bis der Innendruck von anfänglich 3 bar auf 1 bar gefallen war. Das danach aufgenommene Gaschromatogramm der flüchtigen Anteile des Autoklaveninhalts (Bedingungen wie in Beispiel 9) zeigte, daß zu 99 % eine Substanz enthalten war, die durch ¹H- und ¹⁹F-Kernresonanzspektren als 2.2-Dihydro-octafluorbutan identifiziert wurde.

### Beispiel 13

In einem Rührkolben aus Borsilikatglas wurden 79 g (ca. 0,5 Mol) des flüssigen Hydrofluorids [(n-C₂H₅)₃N · 2,8 HF] vorgelegt und 150 g Perfluor-(2-methyl-2-penten) (0,5 Mol) aus einem Tropftrichter bei 25°C zugegeben. Danach wurden 250 ml Wasser zugesetzt, die organische Phase abgetrennt (Ausbeute 155 g) und über Magnesiumsulfat getrocknet. Sie bestand gemäß Gaschromatogramm zu 96,1 % aus einem Produkt, das mittels ¹H- und ¹³F-Kernresonanzspektren als 2-H-Perfluor-(2-methylpentan) identifiziert wurde.

### Beispiel 14

In einem 500 ml-Rührkolben aus Borsilikatglas wurden 120 g (ca. 0,5 Mol) des flüssigen Hydrofluorids [(n-C₄H₉)₃N · 2,6 HF] vorgelegt und 150 g Perfluor-(2-methyl-2-buten) (0,5 Mol) aus einem Tropftrichter bei 25°C zugegeben. Das homogene Reaktionsgemisch wurde sodann destilliert. Man erhielt 150 g eines Produktes vom Siedepunkt 60 bis 61°C, welches mittels ¹H- und ¹⁹F-NMR-Spektren als 2-H-Perfluor-(2-methylpentan) identifiziert wurde.

### Beispiel 15

In einem Rührautoklaven von 300 ml Fassungsvermögen wurden 72,5 g (0,5 Mol) des Hydrofluorids [Piperidin · 2,9 HF] vorgelegt und 30 g Hexafluorpropen (0,2 Mol) aus einem Vorrats-Druckbehälter eingepreßt. Das Reaktionsgemisch wurde 24 h bei 60° C gerührt. Das danach aufgenommene Gaschromatogramm der flüchtigen Anteile des Reaktorinhalts zeigte einen Gehalt von 88,7 % 2-H-Heptafluorpropan neben 11,1 % nicht umgesetztem Hexafluorpropen an.

### Beispiel 16

In einem Rührautoklaven von 300 ml Fassungsvermögen wurden 71 g (0,5 Mol) des Hydrofluorids [Morpholin · 2,8 HF] vorgelegt und 30 g (0,2 Mol) Hexafluorpropen aus einem Vorrats-Druckbehälter eingepreßt. Das Reaktionsgemisch wurde 24 h bei 60°C gerührt. Das danach aufgenommene Gaschromatogramm der flüchtigen Anteile des Autoklaveninhalts zeigte einen Gehalt von 25 % 2-H-Heptafluorpropan neben ca. 75 % nicht umgesetztem Hexafluorpropen an.

### Beispiel 17

In einem Rührautoklaven von 300 ml Fassungsvermögen wurden 65 g (0,5 Mol) des Hydrofluorids [t-Butylamin · 2,8 HF] vorgelegt und 26 g (0,17 Mol) Hexafluorpropen aus einem Vorrats-Druckbehälter eingepreßt. Das Reaktionsgemisch wurde 24 h bei 60° C gerührt. Das danach aufgenommene Gaschromatogramm der flüchtigen Anteile des Autoklaveninhalts zeigte einen Gehalt von 26 % 2-H-Heptafluorpropan neben 74 % nicht umgesetztem Hexafluorpropen an.

## Patentansprüche

1. Verfahren zur Addition von HF an ein halogeniertes Alken der allgemeinen Formel (I)
R¹CF=CR²R³ (I)
worin R¹ bis R³ folgende Bedeutung haben:
R¹ = F, CF₃ oder CF₂R⁴, mit R⁴ = C₁-C₄-Alkyl, unsubstituiert oder substituiert mit einem oder mehreren Halogenatomen,
R² = H, Halogen oder CF₃,
R³ = H, F, CF₃ oder C₁-C₄-Alkyl, unsubstituiert oder substituiert mit einem oder mehreren Halogenatomen,
dadurch gekennzeichnet, daß man ein halogeniertes Alken der Formel (I) mit mindestens einem Hydrofluorid einer organischen Stickstoffbase der allgemeinen Formel (II) umsetzt,
[B·n HF] (II)
worin B eine organische Stickstoffbase und n eine ganze oder gebrochene Zahl ≤ 4 bedeutet, wobei die Umsetzung von Perfluorisobuten CF₂=C(CF₃)₂ ausgeschlossen sein soll.

2. Verfahren nach Anspruch 1, dadurch gekennzeichnet, daß man ein halogeniertes Alken der allgemeinen Formel (I) einsetzt, bei dem bedeutet:
R¹ = F, CF₃ oder C₂F₅,
R² = F, Cl oder CF₃, und
R³ = H, F oder perfluoriertes C₁-C₄-Alkyl.

3. Verfahren nach Anspruch 1, dadurch gekennzeichnet, daß man als halogeniertes Alken der Formel (I)
CF₂=CF₂, CF₂=CClF, CF₂=CFH, CF₂=CClH, CF₂=CF-CF₃, CF₃-CF=CH-CF₃, 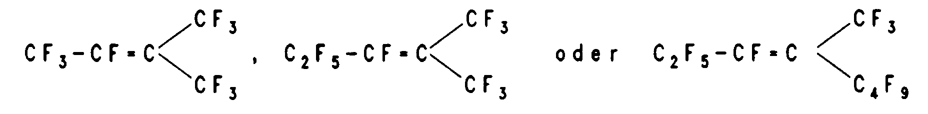 einsetzt.

4. Verfahren nach Anspruch 1, dadurch gekennzeichnet, daß man als halogeniertes Alken der Formel (I) CF₂=CF-CF₃ (Hexafluorpropen) einsetzt.

5. Verfahren nach einem der Ansprüche 1 bis 4, dadurch gekennzeichnet, daß n in der Formel (II) für eine ganze oder gebrochene Zahl von 0,5 bis 3,5 steht.

6. Verfahren nach einem der Ansprüche 1 bis 4, dadurch gekennzeichnet, daß n in der Formel (II) für eine ganze oder gebrochene Zahl von 2 bis 3 steht.

7. Verfahren nach einem der Ansprüche 1 bis 6, dadurch gekennzeichnet, daß B für eine organische Stickstoffbase der Formel (III) R⁴R⁵R⁶N steht, worin die Reste R⁴ bis R⁶ gleich oder verschieden sind und jeder dieser Reste
Wasserstoff,
einen Alkylrest mit 1 bis 20 C-Atomen,
einen Alkenylrest mit 2 bis 20 C-Atomen,
einen Cycloalkylrest mit 5 bis 7 C-Atomen,
einen Cycloalkenylrest mit 5 bis 7 C-Atomen,
einen Aralkylrest mit 7 bis 10 C-Atomen oder
einen Arylrest mit 6 bis 10 C-Atomen bedeutet, der noch mit C₁-C₃-Alkyl- oder C₁-C₃-Alkoxyresten substituiert sein kann,
bedeutet
oder worin zwei der Reste R⁴ bis R⁶ zusammen mit dem sie tragenden N-Atom einen 5 bis 7-gliedrigen Ring bilden, welcher ein O- oder ein weiteres N-Atom enthalten kann,
oder worin die Reste R⁴ bis R⁶ zusammen mit dem sie tragenden N-Atom zwei oder drei 5 bis 7-gliedrige Ringe bilden, welche weitere N-Atome enthalten können.

8. Verfahren nach einem der Ansprüche 1 bis 6, dadurch gekennzeichnet, daß B für eine organische Stickstoffbase der Formel (III) R⁴R⁵R⁶N steht,
worin die Reste R⁴ bis R⁶ gleich oder verschieden sind und jeder dieser Reste
einen Alkylrest mit 1 bis 20 C-Atomen,
einen Cycloalkylrest mit 5 bis 7 C-Atomen,
einen Aralkylrest mit 7 bis 10 C-Atomen oder
einen Arylrest mit 6 bis 10 C-Atomen, der noch mit C₁-C₃-Alkyl- oder C₁-C₃-Alkoxyresten substituiert sein kann,
bedeutet
oder worin zwei der Reste R⁴ bis R⁶ zusammen mit dem sie tragenden N-Atom einen 5 bis 7-gliedrigen Ring bilden, welcher ein O- oder ein weiteres N-Atom enthalten kann,
oder worin die Reste R⁴ bis R⁶ zusammen mit dem sie tragenden N-Atom zwei oder drei 5- bis 7-gliedrige Ringe bilden, welche weitere N-Atome enthalten können.

9. Verfahren nach Anspruch 7 oder 8, dadurch gekennzeichnet, daß mindestens einer der Reste R⁴ bis R⁶ ein Alkylrest mit 1 bis 12 C-Atomen ist.

10. Verfahren nach Anspruch 7 oder 8, dadurch gekennzeichnet, daß mindestens einer der Reste R⁴ bis R⁶ ein Alkylrest mit 1 bis 6 C-Atomen ist.

11. Verfahren nach einem der Ansprüche 1 bis 10, dadurch gekennzeichnet, daß die organische Stickstoffbase B tertiär ist und insgesamt 3 bis 12 C-Atome hat.

12. Verfahren nach einem der Ansprüche 1 bis 6, dadurch gekennzeichnet, daß die organische Stickstoffbase B ein 6-gliedriger heterocyclischer Ring ist, der 1 oder 2 N-Atome enthält und auch benzokondensiert sein kann.

13. Verfahren nach einem der Ansprüche 1 bis 6, dadurch gekennzeichnet, daß die organische Stickstoffbase B ein Trialkylamin mit 3 bis 12 C-Atomen ist.

14. Verfahren nach einem der Ansprüche 1 bis 13, dadurch gekennzeichnet, daß man die Umsetzung bei einer Temperatur von 0 bis 100°C durchführt.

15. Verfahren nach einem der Ansprüche 1 bis 13, dadurch gekennzeichnet, daß man die Umsetzung bei einer Temperatur von 20 bis 80°C durchführt.

16. Verfahren nach einem der Ansprüche 1 bis 15, dadurch gekennzeichnet, daß man die Umsetzung in einer Blasensäule durchführt.

17. Verfahren nach einem der Ansprüche 1 bis 15, dadurch gekennzeichnet, daß man die Umsetzung in einem Druckbehälter durchführt.

## Claims

1. A process for the addition of HF to a halogenated alkene of the formula (I)
R¹CF=CR²R³ (I)
in which R¹ to R³ have the following meaning:
R¹ = F, CF₃ or CF₂R⁴, where R⁴ = C₁-C₄-alkyl, unsubstituted or substituted by one or more halogen atoms,
R² = H, halogen or CF₃,
R³ = H, F, CF₃ or C₁-C₄-alkyl, unsubstituted or substituted by one or more halogen atoms,
which comprises reacting a halogenated alkene of the formula (I) with at least one hydrofluoride of an organic nitrogen base of the formula (II)
[B·n HF] (II)
in which B is an organic nitrogen base and n is an integer or fraction ≤ 4, it being intended that the reaction of perfluoroisobutene CF₂=C(CF₃)₂ is excluded.

2. The process as claimed in claim 1, wherein a halogenated alkene of the formula (I) is employed, in which:
R¹ = F, CF₃ or C₂F₅,
R² = F, Cl or CF₃, and
R³ = H, F or perfluorinated C₁-C₄-alkyl.

3. The process as claimed in claim 1, wherein the halogenated alkene of the formula (I) employed is
CF₂=CF₂, CF₂=CClF, CF₂=CFH, CF₂=CClH, CF₂=CF-CF₃, CF₃-CF=CH-CF₃,

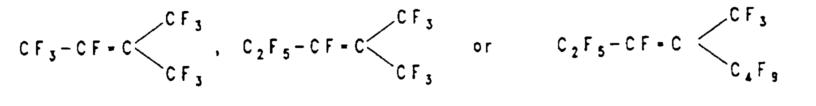

4. The process as claimed in claim 1, wherein the halogenated alkene of the formula (I) employed is CF₂=CF-CF₃ (hexafluoropropene).

5. The process as claimed in one of claims 1 to 4, wherein n in the formula (II) is an integer or fraction from 0.5 to 3.5.

6. The process as claimed in one of claims 1 to 4, wherein n in the formula (II) is an integer or fraction from 2 to 3.

7. The process as claimed in one of claims 1 to 6, wherein B is an organic nitrogen base of the formula (III) R⁴R⁵R⁶N, in which the radicals R⁴ to R⁶ are identical or different and each of these radicals is
hydrogen,
an alkyl radical having 1 to 20 carbon atoms,
an alkenyl radical having 2 to 20 carbon atoms,
a cycloalkyl radical having 5 to 7 carbon atoms,
a cycloalkenyl radical having 5 to 7 carbon atoms,
an aralkyl radical having 7 to 10 carbon atoms or
an aryl radical having 6 to 10 carbon atoms, which can additionally be substituted by C₁-C₃-alkyl or C₁-C₃-alkoxy radicals,
or in which two of the radicals R⁴ to R⁶, together with the nitrogen atom carrying them, form a 5- to 7-membered ring which can contain an oxygen or a further nitrogen atom,
or in which the radicals R⁴ to R⁶, together with the nitrogen atom carrying them, form two or three 5- to 7-membered rings which can contain further nitrogen atoms.

8. The process as claimed in one of claims 1 to 6, wherein B is an organic nitrogen base of the formula (III) R⁴R⁵R⁶N, in which the radicals R⁴ to R⁶ are identical or different and each of these radicals is
an alkyl radical having 1 to 20 carbon atoms,
a cycloalkyl radical having 5 to 7 carbon atoms,
an aralkyl radical having 7 to 10 carbon atoms or
an aryl radical having 6 to 10 carbon atoms, which can additionally be substituted by C₁-C₃-alkyl or C₁-C₃-alkoxy radicals,
or in which two of the radicals R⁴ to R⁶, together with the nitrogen atom carrying them, form a 5- to 7-membered ring which can contain an oxygen or a further nitrogen atom,
or in which the radicals R⁴ to R⁶, together with the nitrogen atom carrying them, form two or three 5- to 7-membered rings which can contain further nitrogen atoms.

9. The process as claimed in claim 7 or 8, wherein at least one of the radicals R⁴ to R⁶ is an alkyl radical having 1 to 12 carbon atoms.

10. The process as claimed in claim 7 or 8, wherein at least one of the radicals R⁴ to R⁶ is an alkyl radical having 1 to 6 carbon atoms.

11. The process as claimed in one of claims 1 to 10, wherein the organic nitrogen base B is tertiary and has a total of 3 to 12 carbon atoms.

12. The process as claimed in one of claims 1 to 6, wherein the organic nitrogen base B is a 6-membered heterocyclic ring which contains 1 or 2 nitrogen atoms and can also be benzo-fused.

13. The process as claimed in one of claims 1 to 6, wherein the organic nitrogen base B is a trialkylamine having 3 to 12 carbon atoms.

14. The process as claimed in one of claims 1 to 13, wherein the reaction is carried out at a temperature from 0 to 100°C.

15. The process as claimed in one of claims 1 to 13, wherein the reaction is carried out at a temperature from 20 to 80°C.

16. The process as claimed in one of claims 1 to 15, wherein the reaction is carried out in a bubble column.

17. The process as claimed in one of claims 1 to 15, wherein the reaction is carried out in a pressure vessel.

## Revendications

1. Procédé d'addition de HF à un alcène halogéné répondant à la formule générale (I)
R¹CF=CR²R³ (I)
dans laquelle R¹ à R³ présentent la signification suivante :
R¹ = F, CF₃ ou CF₂R⁴, avec R⁴ = un groupe alkyle en C₁-C₄, non-substitué ou substitué par un ou plusieurs atomes d'halogène,
R² = H, un atome d'halogène ou CF₃,
R³ = H, F, CF₃ ou un groupe alkyle en C₁-C₄, non-substitué ou substitué par un ou plusieurs atomes d'halogène,
caractérisé en ce qu'on met en réaction un alcène halogéné répondant à la formule (I) avec au moins un hydrofluorure d'une base azotée organique répondant à la formule générale (II),
[B · n HF] (II)
dans laquelle B représente une base azotée organique et n représente un nombre entier ou une fraction ≤ 4, la réaction de perfluoroisobutène CF₂=C(CF₃)₂ devant être exclue.

2. Procédé selon la revendication 1, caractérisé en ce qu'on utilise un alcène halogéné répondant à la formule générale (I), dans laquelle :
R¹ = F, CF₃ ou C₂F₅,
R² = F, Cl ou CF₃, et
R³ = H, F ou un groupe alkyle en C₁-C₄ perfluoré.

3. Procédé selon la revendication 1, caractérisé en ce qu'on utilise à titre d'alcène halogéné répondant à la formule générale (I)
du CF₂=CF₂, du CF₂=CClF, du CF₂=CFH, du CF₂=CClH, du CF₂=CF-CF₃, du CF₃-CF=CH-CF₃,

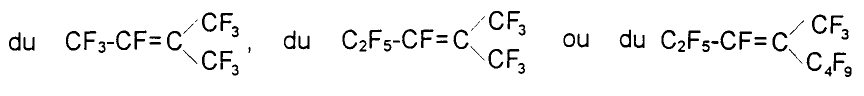

4. Procédé selon la revendication 1, caractérisé en ce qu'on utilise à titre d'alcène halogéné répondant à la formule (I) du CF₂=CF-CF₃ (hexafluoropropène).

5. Procédé selon l'une des revendications 1 à 4, caractérisé en ce que n dans la formule (II) représente un nombre entier ou une fraction de 0,5 à 3,5.

6. Procédé selon l'une des revendications 1 à 4, caractérisé en ce que n dans la formule (II) représente un nombre entier ou une fraction de 2 à 3.

7. Procédé selon l'une des revendications 1 à 6, caractérisé en ce que B représente une base azotée organique répondant à la formule (III) R⁴R⁵R⁶N, dans laquelle les résidus R⁴ à R⁶ sont identiques ou différents et dans laquelle chacun de ces résidus signifie
un atome d'hydrogène,
un résidu alkyle avec 1 à 20 atomes de carbone,
un résidu alcényle avec 1 à 20 atomes de carbone,
un résidu cycloalkyle avec 5 à 7 atomes de carbone,
un résidu cycloalcényle avec 5 à 7 atomes de carbone,
un résidu arylalkyle avec 7 à 10 atomes de carbone ou
un résidu aryle avec 6 à 10 atomes de carbone, qui peut encore être substitué par des résidus alkyles en C₁-C₃ ou alcoxy en C₁-C₃,
ou dans laquelle deux des résidus R⁴ à R⁶, ensemble avec l'atome d'azote qui les porte, forment un cycle à 5 à 7 membres, qui peut contenir un atome d'oxygène ou un atome d'azote supplémentaire,
ou dans laquelle les résidus R⁴ à R⁶, ensemble avec l'atome d'azote qui les porte, forment deux ou trois cycles à 5 à 7 membres, qui peuvent contenir des atomes d'azote supplémentaires.

8. Procédé selon l'une des revendications 1 à 6, caractérisé en ce que B représente une base azotée organique répondant à la formule (III) R⁴R⁵R⁶N, dans laquelle les résidus R⁴ à R⁶ sont identiques ou différents et dans laquelle chacun de ces résidus signifie
un résidu alkyle avec 1 à 20 atomes de carbone,
un résidu cycloalkyle avec 5 à 7 atomes de carbone,
un résidu arylalkyle avec 7 à 10 atomes de carbone ou
un résidu aryle avec 6 à 10 atomes de carbone, qui peut encore être substitué par des résidus alkyles en C₁-C₃ ou alcoxy en C₁-C₃,
ou dans laquelle deux des résidus R⁴ à R⁶, ensemble avec l'atome d'azote qui les porte, forment un cycle à 5 à 7 membres, qui peut contenir un atome d'oxygène ou un atome d'azote supplémentaire,
ou dans laquelle les résidus R⁴ à R⁶, ensemble avec l'atome d'azote qui les porte, forment deux ou trois cycles à 5 à 7 membres, qui peuvent contenir des atomes d'azote supplémentaires.

9. Procédé selon la revendication 7 ou 8, caractérisé en ce qu'au moins un des résidus R⁴ à R⁶ est un résidu alkyle avec 1 à 12 atomes de carbone.

10. Procédé selon la revendication 7 ou 8, caractérisé en ce qu'au moins un des résidus R⁴ à R⁶ est un résidu alkyle avec 1 à 6 atomes de carbone.

11. Procédé selon l'une des revendications 1 à 10, caractérisé en ce que la base azotée organique B est tertiaire et présente en tout 3 à 12 atomes de carbone.

12. Procédé selon l'une des revendications 1 à 6, caractérisé en ce que la base azotée organique B est un noyau hétérocyclique à 6 membres, qui contient 1 ou 2 atomes d'azote et qui peut également être benzocondensé.

13. Procédé selon l'une des revendications 1 à 6, caractérisé en ce que la base azotée organique B est une trialkylamine avec 3 à 12 atomes de carbone.

14. Procédé selon l'une des revendications 1 à 13, caractérisé en ce que la réaction est effectuée à une température de 0 à 100 °C.

15. Procédé selon l'une des revendications 1 à 13, caractérisé en ce que la réaction est effectuée à une température de 20 à 80 °C.

16. Procédé selon l'une des revendications 1 à 15, caractérisé en ce que la réaction est effectuée dans une colonne à bulles.

17. Procédé selon l'une des revendications 1 à 15, caractérisé en ce que la réaction est effectuée dans un récipient sous pression.
